# EUROPÄISCHE PATENTSCHRIFT

(11) **EP 2 349 039 B1**
(45) Veröffentlichungstag und Bekanntmachung des Hinweises auf die Patenterteilung: **17.08.2016**
(21) Anmeldenummer: 09775634.0
(22) Anmeldetag: 16.09.2009
(51) Int. Cl.: A61B 17/72, A61B 17/80, A61B 17/17

(54) **IMPLANTAT, INSBESONDERE MARKNAGEL FÜR DIE BEHANDLUNG EINER PROXIMALEN HUMERUSFRAKTUR**
IMPLANT, IN PARTICULAR INTRAMEDULLARY PIN FOR TREATING A PROXIMAL FRACTURE OF THE HUMERUS
IMPLANT, EN PARTICULIER CLOU MÉDULLAIRE, POUR LE TRAITEMENT D'UNE FRACTURE DE L'HUMÉRUS PROXIMAL

(30) Priorität: 16.09.2008 AT 14442008
(43) Veröffentlichungstag der Anmeldung: 03.08.2011
(73) Patentinhaber: Haininger, Christian, 2540 Bad Vöslau (AT)
(72) Erfinder: Haininger, Christian, 2540 Bad Vöslau (AT)
(74) Vertreter: Sonn & Partner Patentanwälte
(86) Internationale Anmeldenummer: PCT/AT2009/000358
(87) Internationale Veröffentlichungsnummer: WO 2010/031098

(56) Entgegenhaltungen:
- EP-A- 1 398 000
- EP-A- 1 685 803
- WO-A-97/39693
- WO-A-2009/021624
- DE-A1- 19 912 696
- DE-U1- 9 115 200
- US-A- 5 658 287
- US-A- 5 766 174
- US-B1- 7 179 259

## Beschreibung

Die Erfindung betrifft ein Implantat, insbesondere Marknagel für die Behandlung einer proximalen Humerusfraktur, mit wenigstens einem distalen und wenigstens einem proximalen Teil, die zur Veränderung der Implantatlänge axial relativ zueinander verschiebbar angeordnet sind und miteinander zusammenwirkende Führungsflächen aufweisen.

Die Fraktur des proximalen Anteiles des Humerus, also der Bruch des Oberarmkopfes in zwei oder mehrere Teile und/oder des angrenzenden Oberarmschaftes, ist noch immer ein nicht endgültig gelöstes Probleme der Frakturversorgung in der Unfallchirurgie. Dies wird auch durch die Vielzahl an verschiedenen Vorgehensweisen, Operationsmöglichkeiten und Implantaten, die seit Jahren in diesem Zusammenhang diskutiert und empfohlen werden, gezeigt.

Die Schulter ist das beweglichste Gelenk des menschlichen Körpers. Um das zu ermöglichen, ist die Gelenkspfanne des Schultergelenks kaum ausgebildet. Die Führung bei der Bewegung und die Stabilität werden nur durch die Gelenkskapsel, Bänder, Muskulatur und Sehnen, die direkt am Oberarmkopf oder in unmittelbarer Umgebung ansetzen, erreicht.

Bei einem Bruch des Oberarmkopfes in zwei, meisten aber in mehrere Teile kommt es typischerweise zu folgender Situation: Die Bruchzone zwischen Oberarmkopf und Oberarmschaft (subkapitale Frakturzone) ist sehr instabil, die Bruchstücke sind oft beträchtlich verschoben. Die Teile des Oberarmkopfes, an denen Sehnen ansetzen (Tuberkula), werden nicht nur durch das Trauma sondern auch durch den Zug der Sehnen disloziert. Das Gelenkflächen tragende Stück ist wegen der fehlenden Abstützung abgekippt. Ist der angrenzende Teil des Oberarmschaftes ebenfalls betroffen, kann auch hier eine Verschiebung von Bruchstücken stattfinden. Auf jeden Fall kommt es aber zu einer weiteren Zunahme der Instabilität. Hinzu kommt, dass das Tuberkulum majus, an dem die für die Bewegung des Oberarmes wesentlichen Sehnen ansetzen und das zusätzlich eine wichtige Abstützung des Gelenkfläche tragenden Stückes bietet, fast immer in mehrere, teils sehr kleine Stücke zerbricht.

Außerdem ist die Fraktur des Oberarmkopfes typischerweise eine Verletzung, die in erster Linie beim älteren Menschen vorkommt. In Folge der schlechteren Qualität des Knochens finden hier Implantate oft nur ungenügend Halt. Eine vollkommene Ruhigstellung des Schultergelenkes in einem Verband ist nicht möglich, daher kommt es auch nach einer operativen Versorgung noch immer zum Auftreten von Zug-, Biegungs- und Scherkräften auf die subkapitale Frakturzone und auf Teile des Oberarmkopfes bzw. des Oberarmschaftes.

In den Wochen nach dem Trauma, wenn der Patient wieder beginnt, den Arm zu bewegen, kommt es immer zu einem "Zusammensinken" der subkapitalen Frakturzone. Dies ist die Voraussetzung, dass die Fraktur zusammenwächst. Nur dadurch sind eine schnelle Frakturheilung und die Vermeidung einer Pseudoarthrose möglich. Es wirken weiterhin Zugkräfte der Sehnen (Rotatorenmanschette) auf Teile des Oberarmkopfes ein. Wenn Schrauben von Implantaten keinen guten Halt im Knochen finden oder wenn nicht alle Knochenstücke durch die Implantate bewegungsstabil fixiert sind, besteht die Gefahr, dass es in den Wochen nach der Operation, wenn die Muskulatur wieder verwendet wird, zu einer sekundärer Dislokation von Frakturteilen und/oder Osteosynthesematerial kommt.

Die Osteosynthese sollte bewegungsstabil sein, um möglichst frühzeitig ohne die Gefahr der sekundären Dislokation mit der physikalischen Therapie, beginnen zu können. Gerade beim älteren Menschen ist eine längere Ruhigstellung des Schultergelenkes mit einer Schrumpfung der Gelenkskapsel und damit mit einer oft schmerzhaften Bewegungseinschränkung verbunden.

Eine Entfernung des Implantates sollte nicht planmäßig notwendig sein, da es durch eine weitere Operation zur Schädigung von Weichteilen kommen kann. Außerdem wird durch die neuerlich notwendige postoperative Schonung die Rehabilitation negativ beeinträchtigt.

Die derzeit gängigen Implantate, die zur Versorgung dieser Frakturen verwendet werden, lassen sich in zwei Gruppen einordnen:
Semirigide Implantate, die primär als Ziel haben, die subkapitale Frakturzone im reponierten Zustand zu fixieren, und die gleichzeitig ein Zusammensinken der Fraktur zulassen. Die restlichen Bruchstücke des Oberarmkopfes werden dabei mit Schrauben fixiert. Die Vorteile dieser Methode liegen im guten knöchernen Verheilen der subkapitalen Fraktur, da durch das Zusammensinken eine gewisse Kompression entsteht, die die Frakturheilung fördert. Als Nachteil zeigt sich, dass sich die verschraubten Tuberkula in den ersten Wochen noch leicht verschieben können. Dies einerseits, weil es nicht möglich ist, alle Bruchstücke mittels Schrauben zu fixieren, andererseits, weil die Schrauben in der weichen Knochenstruktur des Oberarmkopfes nur sehr wenig Halt finden und ein Durchschrauben der gegenüberliegenden, die Gelenkfläche tragenden harten Kortikalis vermieden werden muss, da die Schraubenspitzen dann ins Gelenk ragen würden. Bei einer Dislokation des Tuberkulum majus fehlt aber auch das Widerlager des Gelenkfläche tragenden Teiles, wodurch ein Abkippen des ganzen Oberarmkopfes in den ersten Tagen und Wochen nach der Operation möglich ist. Daher müssen Frakturen, die mit diesen Implantaten versorgt werden, oft längere Zeit mit einem Verband fixiert werden, wodurch es zur Bewegungseinschränkung des Schultergelenkes kommt. Frakturen, die zusätzlich auch in den Oberarmschaft reichen, können mit diesen Implantaten meist gar nicht versorgt werden. Zu den semirigiden Implantaten zählen:
   - Spongiosaschrauben, Bohrdrähte
      Die Schrauben bzw. Bohrdrähte können gut in idealer Stellung, das heißt in divergierender Lage und im rechten Winkel auf die zu erwartenden einwirkenden Kräfte, positioniert werden. Sie finden aber oft nur schlecht in der Knochenspongiosa des Oberarmkopfes Halt. In Folge der schlechten Verankerungsmöglichkeiten kommt es bei Bewegungen im Schultergelenk oft zum Verrutschen der Bruchstücke und der Schrauben bzw. der Drähte. Beim Zusammensinken der subkapitalen Fraktur können die Implantate nach lateral "zurückrutschen" wodurch das Gelenksflächen tragende Fragment wieder abkippen kann.
   - Bohrdrähte und Humerusblock
      Der Block verhindert das Zurückrutschen der Bohrdrähte. Beim Zusammensinken kann es zum Perforieren der Bohrdrähte durch die Gelenksfläche in das Gelenk kommen. Dieses Implantat muss immer nach einigen Wochen in einer zweiten Operation entfernt werden. Die Tuberkula werden mit Schrauben versorgt, für welche die oben beschriebenen Nachteile gelten.
   - Markdrähte
      Oft ist es nicht möglich, die Fraktur zu reponieren bzw. das reponierte Ergebnis zu fixieren. Auch hier kann es leicht zum Rutschen der Drähte, entweder proximal durch die Gelenksfläche oder auch nach distal kommen. Auch hier werden die Tuberkula mit Schrauben versorgt.
   - Helix wire
      Der Helix wire kann das Zusammensinken der subkapitalen Frakturzone gut kompensieren, es kommt sehr selten zur Perforation durch die Gelenksfläche. Es gelten jedoch dieselben Nachteile wie bei den Markdrähten; es ist bei diesem Implantat auch oft nicht möglich, die Rotation zu fixieren.

### Rigide Implantate

Mit diesen Implantaten kann meist ein gutes Repositionsergebnis erzielt werden. Auch ist die Fixation der Tuberkula meist besser möglich. Einerseits, weil die Bruchstücke des Tuberkulum majus durch die Fläche der Platte fixiert wird, andererseits, weil die Schrauben nicht nur im spongiösen Knochen fixiert werden, sondern auch im Implantat (Platte, Nagel) Halt finden. Allerdings können diese Implantate nicht die postoperativ auftretenden Bewegungen kompensieren. Im Bereich der subkapitalen Frakturzone ist eine Kompression nicht möglich, es kann daher hier zu mangelnder knöcherner Heilung und zu Pseudoarthrosen kommen. Wird der Arm vor der knöchernen Heilung bewegt, können Teile der Implantate, die ja am Oberarmschaft fixiert sind, beim Zusammensinken der Fraktur durch die Gelenksfläche perforieren oder bewirken, dass die Frakturstücke des Oberarmkopfes neuerlich verrutschen.
- Platte
   Die Schrauben der Platte finden zwar guten Halt am Oberarmschaft, jedoch wenig Halt im Knochen des Oberarmkopfes. Bei der postoperativen Bewegung kommt es oft zur Dislokation oder zum Ausreißen dieser Schrauben, wodurch das Repositionsergebnis verloren geht und die Platte wieder entfernt werden muss. Außerdem wird durch die flächenhafte Kompression der Platte an den Knochen die Durchblutung des Periosts und damit die Knochenheilung gestört.
- Winkelstabile Platte
   Die Schrauben sind in der Platte winkelstabil fixiert und können nicht verrutschen. Allerdings finden sie im spongiösen Knochen trotzdem keinen guten Halt. Vor allem das Gelenkflächen tragende Bruchstück des Oberarmkopfes kann sich von den Spongiosaschrauben der Platte "lösen" und neuerlich abkippen. Bei guter Einheilung sowohl in den Oberarmkopf als auch in den Oberarmschaft kann es bei mangelnder subkapitaler Heilung in Folge der Bewegungen in dieser Zone zum Plattenbruch kommen. Zur Implantation von Platten ist ein relativ großer Hautschnitt mit den damit verbundenen Nachteilen der Weichteilschädigung notwendig.
- Humerusnagel
   Als intramedullärer Kraftträger kompensiert der Nagel alle auftretenden Kräfte gut, es kommt auch nie zum Nagelbruch. Der Operationszugang ist klein, schont die Weichteile und die Frakturzone wird nicht zusätzlich traumatisiert. Allerdings ist der Nagel sowohl im Oberarmkopf als auch am Oberarmschaft fixiert und erlaubt im subkapitalen Frakturbereich kein Zusammensinken. Es kann daher vorkommen, dass beim Zusammensinken der subkapitalen Frakturzone der Oberarmkopf oder Teile davon über den Nagel nach caudal verlagert werden und somit der Nagel oder die Verriegelungsschrauben über den Knochen hinausragen. Können die Schrauben den Oberarmkopf gut fixieren, verhindert der Nagel ein Zusammengleiten der subcapitalen Frakturzone. Damit fehlt die Kompression der Bruchflächen und es kann zu fehlender Bruchheilung mit anhaltenden Bewegungsschmerzen kommen.
- Humerusnagel in Verbindung mit winkelstabilen Platten (WO 2009/021624 A1)
   Die Nachteile des intramedullären Nagels (Gefahr der Dislokation des Tuberkulum minus, wenige Schrauben zur Fixation des Gelenkfläche tragenden Bruchstückes) werden durch die Verwendung einer kleinen Platte, die das Tuberkulum majus fixiert und die mittels einer starren Verbindung am Nagel fixiert ist, minimiert. Die Schrauben im Oberarmkopf werden in der Platte winkelstabil fixiert. Der Vorteil ist zweifellos, dass das Tuberkulum majus deutlich besser fixiert ist.
   Als Nachteil bleibt aber, dass, wie bei allen verwendeten Schrauben, ein Fassen des Schraubengewindes in der gegenüberliegenden dünnen Korticalis nur begrenzt möglich ist, sodass eine Dislokation des Gelenkfläche tragenden Frakturstückes weiterhin möglich ist. Da die Platte starr mit dem Nagel verbunden ist, kann es beim Zusammensinken der Fraktur in der subcapitalen Zone in gleicher Weise wie beim Humerusnagel zu einer Dislokation der gesamten Nagel/Platten-Konstruktion nach cranial kommen. Dabei kann es durch das relative Höhertreten der Platte zum "Impingementsyndrom" kommen bzw. im schlechtesten Fall zu einer Perforation der Nagel-Platten-Konstruktion durch den Oberarmkopf nach cranial.

Hin und wieder kann es, unabhängig vom Operationsergebnis, notwendig werden, in späterer Folge einen operierten Humeruskopf durch eine Prothese zu ersetzen. Gemäß dem Stand der Technik muss hierzu ein gegebenenfalls vorhandenes Implantat, welches der Reposition und Fixierung einer Humerusfraktur dient, entfernt werden, was sich häufig als schwierig herausstellt, wenn das Implantat fest in den Knochen eingewachsen ist. Eine entsprechende Operation geht hier mit einer weitreichenden Beeinträchtigung des Knochens sowie mit einer beträchtlichen Weichteilirritation einher, sodass das erfolgreiche Einsetzen eines Implantats erschwert wird. Es wäre somit wünschenswert, einen Teil eines eingesetzten Implantats als Schaft für eine Prothese nutzen zu können.

Aus DE19912696 ist ein Implantat mit den Merkmalen des Oberbegriffs von Anspruch 1 bekannt.

Es ist Aufgabe der vorliegenden Erfindung, ein Implantat der eingangs genannten Art dahingehend weiterzubilden, dass eine möglichst optimale Kombination der Vorteile der oben dargestellten Implantate gelingt, wobei die Nachteile der einzelnen Varianten vermieden werden sollen. Die Frakturstücke des Oberarmkopfes sollen nach Reposition im Sinne einer proximalen Verriegelung am Implantat fixiert werden. Das Gelenkfläche tragende Bruchstück soll mittels Schrauben, Bohrdrähten oder Stiften fixiert bzw. abgestützt werden. Diese dürfen die gegenüberliegende Knochengrenze (d.h. die Gelenksfläche) nicht überschreiten, sollen jedoch dieses Gelenkflächen tragende Knochenstück sowohl gegen Druck, als auch gegen tangentiale Scherkräfte abstützen.

Zusätzlich sollen die Tuberkula in ihrer anatomischen Position fixiert und bis zum knöchernen Anheilen gehalten werden. Die Fixation muss wegen der oft schlechten Knochenqualität winkelstabil am Implantat verankert sein, um eine sekundäre Dislokation zu vermeiden.

Bei Frakturen des Tuberkulum majus in mehrere Teile sollte eine flächenhafte Fixation erreicht werden.

Um die Rotation zu fixieren, ist eine distale Verriegelung in typischer Weise notwendig. Liegt auch eine Fraktur des Oberarmschaftes vor, sind verschiedene Nagellängen notwendig, um eine ausreichende intramedulläre Schienung zur ermögLichen. Liegen verschobene Frakturstücke am Oberarmschaft vor, sollten auch diese mittels Schrauben in der entsprechenden Höhe am Nagel fixiert, werden. Im Bereich der subkapitalen Frakturzone soll das "Zusammensinken" der Frakturzone über eine vorgegebene Maximaldistanz in den Wochen nach der Operation möglich sein, ohne dass die Rotationsstabilität oder die Fixation der restlichen Frakturstücke beeinträchtigt wird.

Das Operationstrauma sollte so gering wie möglich gehalten werden. Eine Entfernung des Implantates sollte nicht zwingend notwendig sein.

Weiters ist es Aufgabe der vorliegenden Erfindung, ein Implantat der eingangs genannten Art dahingehend weiterzubilden, dass eine spätere Verwendung eines Teils des Implantats als Prothesenschaft möglich wird.

Zur Lösung dieser Aufgaben ist das Implantat der eingangs genannten Art erfindungsgemäß dahingehend weitergebildet, dass der proximale und der distate Teil miteinander zusammenwirkende Anschläge zur Begrenzung der axialen Relativverschiebung aufweisen, dass der proximale und der distale Teil innerhalb der durch die Anschläge definierten Begrenzungen nach einer Operation relativ zueinander frei verschiebbar sind, so dass ein Zusammensinken einer subkapitalen Frakturzone innerhalb der durch die Anschläge definierten Grenzen ermöglicht ist, dass der proximale und der distale Teil jeweils mit wenigstens einer Querbohrung zur Aufnahme und/oder Festlegung von Fixationsmitteln versehen sind und dass Mittel zum Verriegeln der Relativverdrehung der beiden Teil um die Implantatachse vorgesehen sind. Dadurch, dass der proximale und der distale Teil miteinander zusammenwirkende Anschläge zur Begrenzung der axialen Relativverschiebung aufweisen, und dass der proximale und der distale Teil innerhalb der durch die Anschläge definierten Begrenzungen relativ zueinander frei verschiebbar sind, wird ein Zusammensinken der subkapitalen Frakturzone innerhalb der durch die Anschläge definierten Grenzen ermöglicht. Bei einem Zusammensinken der subkapitalen Frakturzone während des Heilungsprozesses bewegen sich der proximale und der distale Teil des Implantats aufeinander zu, sodass ein Heraustreten des Marknagels oder von Fixationsmitteln aus dem proximalen Ende des Humerus vermieden wird und eine optimale Erhaltung des Repositionsergebnisses erreicht wird. Die axiale Verschiebbarkeit ist hierbei durch Anschläge in beide Richtungen begrenzt, sodass ein Zusammensinken der subkapitalen Frakturzone nur innerhalb der vorgegebenen Grenzen erfolgen kann. Innerhalb dieser Grenzen sind die Teile aber frei verschiebbar, und es sind insbesondere keine gesonderten, die Verschiebbarkeit ggf. von außen freigebenden oder einstellenden Verriegelungs-, Freigabe- bzw. Einstellmittel oder dgl. vorgesehen.

Gleichzeitig kann dadurch, dass der proximale und der distale Teil jeweils mit wenigstens einer Querbohrung zur Aufnahme und/oder Festlegung von Fixationsmitteln versehen sind, eine Fixierung des Marknagels im Humerus sowohl im Schaft- als auch im Kopfbereich durchgeführt werden, wobei weitere Frakturstücke mittels weiterer Schrauben wie bei einer Platte in der gewünschten Position fixiert werden können. Dadurch, dass Mittel zum Verriegeln der Relativverdrehung der beiden Teile um die Implantatachse vorgesehen sind, ist sichergestellt, dass bei freier Verschieblichkeit der proximalen und distalen Teile zueinander das Repositionsergebnis einer subkapitalen Fraktur nicht gefährdet ist.

Mit Vorteil ist das erfindungsgemäße Implantat dahingehend weitergebildet, dass die Führungsfläche des distalen Teils an einem mit verringertem Durchmesser ausgebildeten vorstehenden Abschnitt desselben ausgebildet ist und dass die Führungsfläche des proximalen Teils am Innenumfang eines den vorstehenden Abschnitt des distalen Teils umgreifenden hohlen Abschnitts ausgebildet ist. Dies gestattet eine besonders einfache und kompakte Bauweise des erfindungsgemäßen Implantats, wobei in besonders einfacher Weise der distale Teil in den als Hülse ausgebildeten proximalen Teil eintaucht.

Mit Vorteil ist das erfindungsgemäße Implantat dahingehend weitergebildet, dass die Führungsfläche der Hülse an einem mit verringertem Innendurchmesser ausgebildeten Bereich ausgebildet ist, wodurch die Hülse im Bereich ihrer Führungsfläche und somit im Bereich des mechanischen Zusammenwirkens mit dem distalen Teil des Implantats mit einer verstärkten Wanddicke ausgebildet ist. Dadurch wird eine erhöhte Stabilität des gesamten Implantats erreicht. Weiters ergibt sich auf Grund des Bereichs mit verringertem Innendurchmesser in der Hülse eine ringförmige Fläche, die als Anschlag für die Begrenzung der relativen Verschiebbarkeit der beiden Implantatteile dienen kann.

Mit Vorteil ist die Erfindung in diesem Zusammenhang dahingehend weitergebildet, dass ein am bzw. im freien Ende des vorstehenden Abschnitts des distalen Teils verstellbar festlegbarer Anschlagbolzen vorgesehen ist und dass der mit verringertem Innendurchmesser ausgebildete Bereich der Hülse einen ringförmigen Vorsprung als Anschlagfläche für den Anschlagbolzen bildet. Auf diese Weise kann der mögliche Verschiebeweg der beiden Teile zueinander einfach eingestellt werden.

In besonders vorteilhafter Weise ist das erfindungsgemäße Implantat dahingehend weitergebildet, dass der distale Teil einen über den Außendurchmesser des proximalen Teils vorstehenden Anschlag für das Angreifen eines Zielgeräts aufweist. An einem solchen Anschlag kann ein Zielgerät unter gleichzeitiger Fixierung des verschieblichen proximalen Teils angreifen, sodass das Implantat beim Einsetzen in den Oberarm, was häufig durch Einschlagen geschieht, gegen eine relative Verschiebung der beiden Teile zueinander gesichert ist und somit nach dem Einsetzen der maximale Verschiebeweg für das erwünschte Zusammensinken der Fraktur zur Verfügung steht.

Mit Vorteil weist der proximale Teil wenigstens zwei, bevorzugt eine Mehrzahl einander kreuzender Querbohrungen auf, in welche Knochenschrauben eingebracht werden können, um eine Mehrzahl von Bruchstücken am Implantat festlegen zu können.

In einfacher Weise ist das Implantat gemäß der vorliegenden Erfindung dahingehend weitergebildet, dass die Führungsflächen abschnittsweise zylindrisch ausgebildet sind, wobei die Ausbildung mit Vorteil dahingehend weitergebildet sein kann, dass die Führungsflächen einen von einem kreisrunden Querschnitt abweichenden Querschnitt des Implantats bilden. Wenn die Führungsflächen abschnittsweise zylindrisch ausgebildet sind, kann die Fertigung des Implantats in einfacher und kostengünstiger Weise erfolgen, wobei dann, wenn die Führungsflächen einen von einem kreisrunden Querschnitt abweichenden Querschnitt aufweisen, unmittelbar eine Verdrehsicherung der beiden Teile zueinander erzielt wird. Das Implantat kann dabei in bevorzugter Weise dahingehend weitergebildet sein, dass die Führungsflächen jeweils wenigstens eine in axialer Richtung verlaufende Nut aufweisen, in deren gemeinsamem Querschnitt ein Stab aufgenommen ist. Mit einer solchen Ausbildung kann einerseits die Fertigung kostengünstig erfolgen, wobei die gewünschte Verdrehsicherung der beiden Teile zueinander durch den in die Nuten eingelegten Stab erfolgt.

Die Verdrehsicherung kann bevorzugt auch dadurch erfolgen, dass der proximale oder der distale Teil ein sich in axialer Richtung erstreckendes Langloch aufweist, in dem ein am anderen Teil rotationsfest angeordneter Bolzen in axialer Richtung geführt ist.

Als Fixationsmittel kann im Rahmen der Erfindung wenigstens eine Knochenschraube vorgesehen sein. Der bzw. die Knochenschraube(n) kann (können) dabei in Querbohrungen im distalen Teil des Implantats vorgesehen sein, um den Nagel entsprechend im Knochen festzulegen. Im proximalen Teil kann ebenfalls wenigstens eine in einer Querbohrung des Implantats aufgenommene Schraube vorgesehen sein.

In vielen Fällen ist es aber nicht möglich, die gesamte Trümmerzone mit einzelnen Fixationsschrauben zu fixieren. Um hier Abhilfe zu schaffen, sieht eine bevorzugte Weiterbildung des Implantats vor, dass als Fixationsmittel eine Platte vorgesehen ist, die relativ zum proximalen Teil des Implantats festlegbar ist. Mit der Platte gelingt es die Bruchstücke flächenhaft zu fixieren.

Zur Festlegung der Platte ist bevorzugt wenigstens eine Schraube vorgesehen, welche die Platte mit dem proximalen Teil des Implantats verbindet. Der Abstand zwischen der Platte und dem Implantat muss dabei nicht vorgegeben sein, sondern es wird die Platte bevorzugt durch die Schraube zu dem Implantat hingezogen, bis sie die Knochengrenze erreicht hat.

Um eine Rotationsstabilität der Platte relativ zum Implantat zu erreichen, ist zusätzlich zur Schraube bevorzugt ein Stift zum Verbinden der Platte mit dem Implantat vorgesehen, dessen Schraubenkopf ein Gewinde zum Einschrauben in die Platte aufweist und dessen dem Schaubenkopf abgewandtes Ende in eine Querbohrung des Implantats einführbar ist. Dadurch wird ein Abstehen der Platte und damit ein Impingementsyndrom vermieden. Die Platte ist mit Vorteil dahingehend ausgebildet, dass sie der Krümmung der Knochenteile, die es zu fixieren gilt, nachgebildet ist. Ferner weist sie vorspringende Leisten oder Spitzen auf, die an den Bruchstücken Halt finden, wodurch einerseits eine sekundäre Dislokation einzelner Knochenstücke vermieden wird. Andererseits wird eine flächenhafte Auflage der Platte vermieden und damit die Durchblutung des Knochens nicht gestört (Periostkompression).

Zur weitern Fixierung der Knochenstücke ist die Ausbildung bevorzugt derart getroffen, dass die Platte eine Mehrzahl von Durchbrechungen zur Aufnahme von Fixationsschrauben und/oder Stiften zum Fixieren von Knochenfragmenten aufweist. Die zum Fixieren von Knochenfragmenten vorgesehenen Stifte und/oder Fixationsschrauben sind dabei mit Vorteil winkelstabil in der Platte festlegbar.

Die Fixationsschrauben bzw. Knochenstifte werden vor allem zur Abstützung des Gelenkfläche tragenden Teiles des Oberarmkopfes verwendet. Die Knochenstifte können in verschiedenen Längen ausgebildet sein und eine Spitze aufweisen, die an der gegenüberliegenden Korticalis gut verankert werden kann. Die Stifte können relativ zueinander divergierende Lage haben, können teilweise auch durch den Nagel verlaufen, wofür Bohrungen im Nagel vorgesehen sein können. Dadurch können sie den Gelenkfläche tragenden Teil des Oberarmkopfes gegen alle einwirkenden Kräfte, unabhängig von der Stellung des Oberarmkopfes zur Pfanne, abstützen. Wenn die Stifte in der Platte winkelstabil fixiert werden, wirken sie gleich einer Verriegelungsschraube als Mittel zum Verriegeln der Relativverdrehung der beiden Teile um die Implantatachse.

Für das Implantieren der Erfindung vorteilhaft ist eine Zielvorrichtung zum Setzen von Knochenschrauben in Querbohrungen des erfindungsgemäßen Implantats, wobei die Zielvorrichtung dadurch gekennzeichnet ist, dass die Zielvorrichtung zwei in axialer Richtung relativ zueinander verschiebbare Teile aufweist, von denen der eine Teil mit dem proximalen oder dem distalen Teil des Implantats verbindbar und gemeinsam mit diesem relativ zum anderen Teil verschiebbar ist.

Eine solche Zielvorrichtung dient zum einen in herkömmlicher Weise dazu, an geeigneten Stellen Bohrungen am Knochen anzubringen, um entsprechende Schrauben im Knochen und am Implantat festzulegen. Dadurch allerdings, dass die Zielvorrichtung zwei in axialer Richtung relativ zueinander verschiebbare Teile aufweist, von denen der eine Teil mit dem proximalen oder dem distalen Teil des Implantats verbindbar und gemeinsam mit diesem relativ zum anderen Teil verschiebbar ist, kann jedoch die Zielvorrichtung zum anderen dazu verwendet werden, sicherzustellen, dass das Implantat beim Einsetzen, was üblicherweise durch Einschlagen geschieht, gegen eine relative Verschiebung der beiden Teile zu einander gesichert ist und somit nach dem Einsetzen der maximale Verschiebeweg für das erwünschte Zusammensinken der Fraktur zur Verfügung steht. Erfindungsgemäß ist es hierbei vorgesehen, dass ein Teil der Zielvorrichtung beispielsweise mit dem proximalen Teil des Implantats beispielsweise durch Schrauben verbunden wird, woraufhin dieser Teil durch Feststellmittel so an einem anderen Teil der Zielvorrichtung abstützbar ist, dass ein Verschieben des proximalen Teils gegen den distalen Teil des Implantats verhindert wird.

Mit Vorteil ist die Zielvorrichtung dahingehend weitergebildet, dass der eine Teil der Zielvorrichtung von einer über den proximalen Implantatteil schiebbaren Hülse gebildet ist, die sich an einem Vorsprung des distalen Implantatteils abstützt, und der andere Teil der Zielvorrichtung von einem in der Hülse angeordneten Stab gebildet ist, der mit dem proximalen Implantatteil vorzugsweise mit Hilfe einer Schraubverbindung verbindbar ist, wobei in besonders einfacher Weise, der Stab an seinem proximalen Ende eine Schraubmutter trägt, die sich ggf. mittelbar gegen die Hülse des Zielgeräts stützt, sodass der Teil der Zielvorrichtung, der am proximalen Teil des Implantats festgelegt ist, gegen die Hülse verspannt werden kann, welches sich wiederum am distalen Teil des Implantats abstützt, sodass ein Verschieben der beiden Implantatteile beim Einsetzen ausgeschlossen ist.

Zur Lösung einer weiteren Aufgabe ist es beispielsweise möglich, den distalen Teil des erfindungsgemäßen Implantats als Prothesenschaft für eine Humeruskopfprothese zu verwenden. Hierzu eignet sich das erfindungsgemäße Implantat in besonderer Weise, da es aufgrund des zweiteiligen Aufbaus in einfacher Weise möglich ist, den proximalen Teil des Implantats abzunehmen und eine entsprechende Prothese auf den im Knochen verbliebenen distalen Teil aufzusetzen. Mit Vorteil ist in diesem Zusammenhang vorgesehen, dass der distale Teil ein abnehmbares Zwischenstück trägt. Das abnehmbare Zwischenstück weist dabei die Führungsflächen für die freie axiale Verschiebbarkeit des distalen zum proximalen Teils auf und wird gemeinsam mit dem proximalen Teil abgenommen, sodass lediglich ein Basisabschnitt des distalen Teils im Knochen verbleit, der in der Folge als Prothesenschaft verwendet werden kann. Mit Vorteil wird hierbei so vorgegangen, dass nach der Abnahme des proximalen Teils und des Zwischenteils ein neues Zwischenstück zur Einstellung der Höhen- und Rotationsposition sowie zur Sicherung der Humerusprothese am distalen Teil des Implantats festgelegt wird. Ein solches Zwischenstück fungiert hierbei als Adapterstück, mit welchem eine genaue Positionierung der Humeruskopfprothese möglich ist.

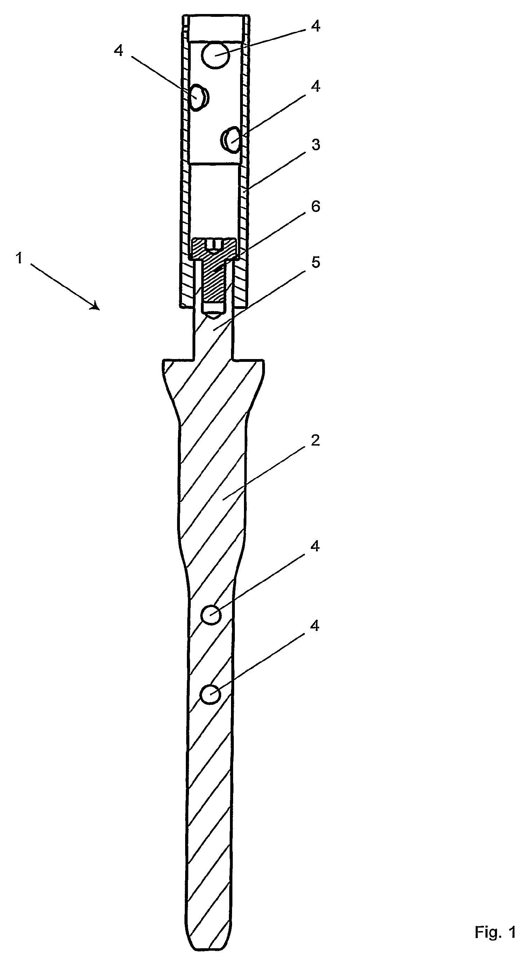

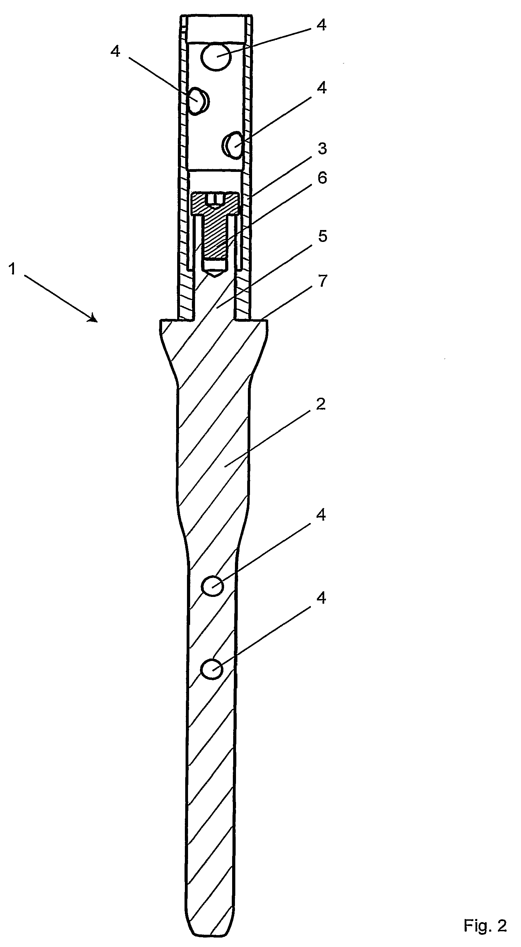

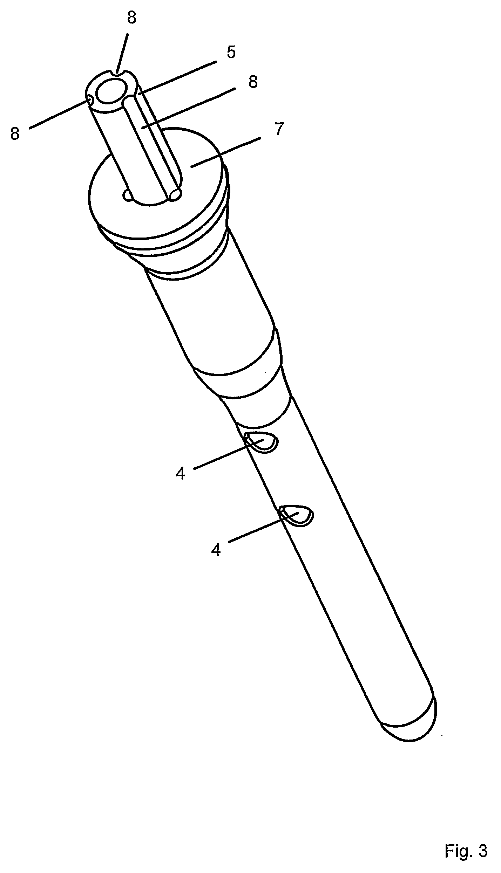

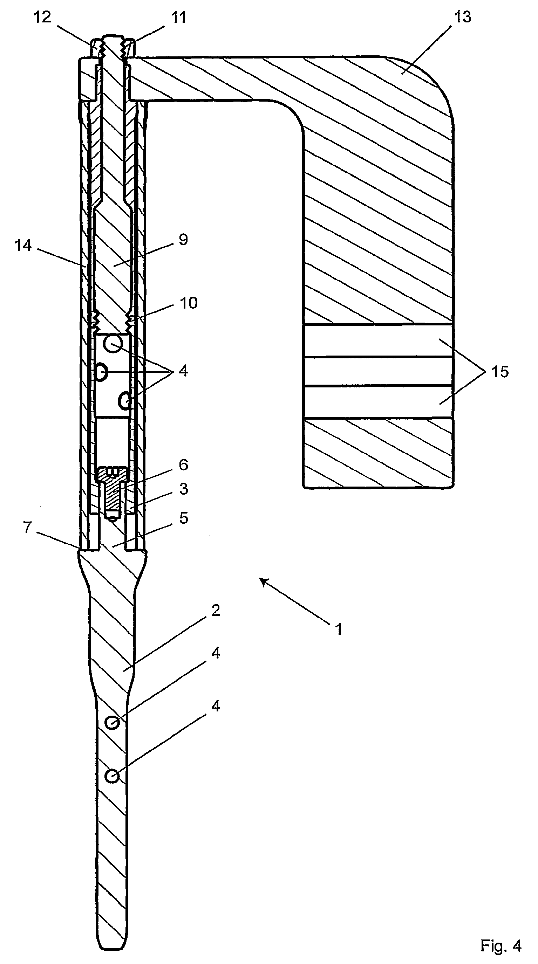

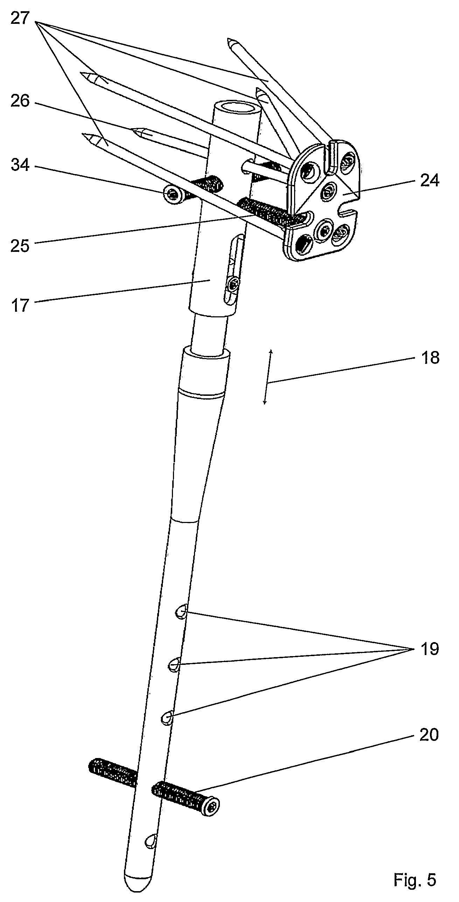

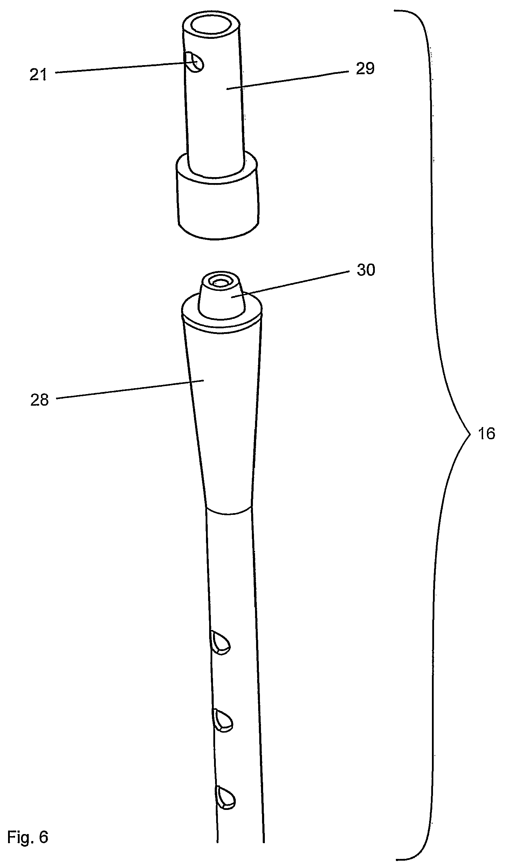

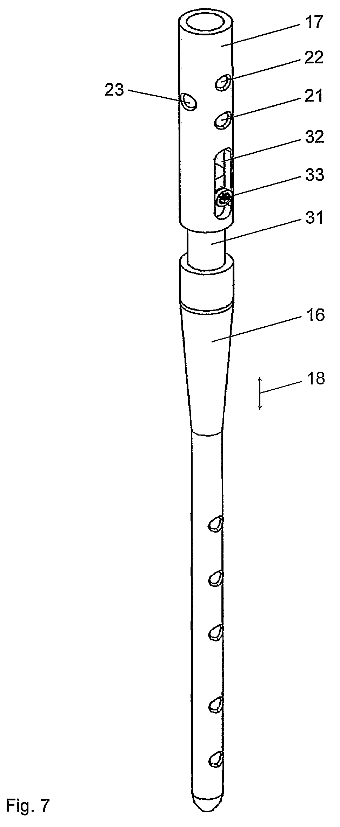

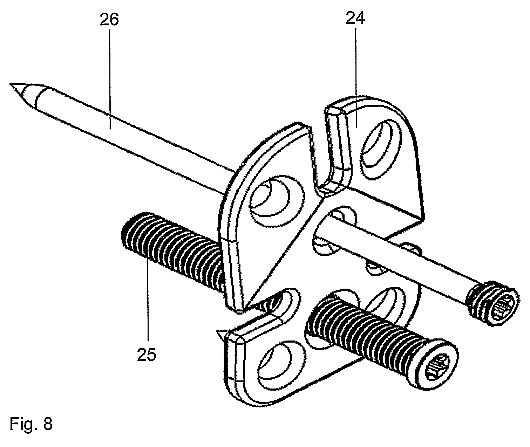

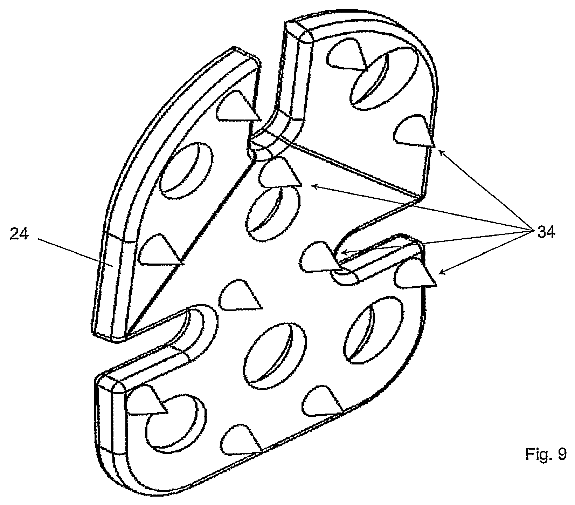

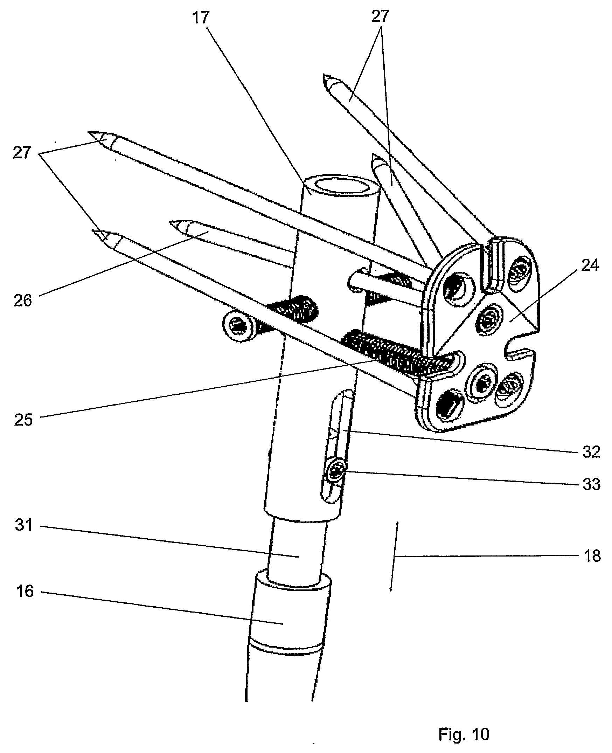

Die Erfindung wird nachfolgend anhand eines in der Zeichnungen näher dargestellten Ausführungsbeispiels näher erläutert. In dieser zeigen Fig. 1 eine erste Ausführungsform des erfindungsgemäßen Implantats, in welcher sich die beiden gegeneinander verschiebbaren Teile in einer ausgezogenen Position befinden, Fig. 2 eine Darstellung des gleichen Implantats, bei welchem die beiden zueinander verschiebbaren Teile in der zusammengesunkenen Position gezeigt sind, Fig. 3 eine perspektivische Darstellung des distalen Teils des Implantats der Fig. 1, bei welchem Nuten zum Einsetzen von Stäben zur Verhinderung der Rotation der beiden verschiebbaren Teile zueinander um die Längsachse des Implantats vorgesehen sind, Fig. 4 eine Darstellung des Implantats der Fig. 1 mit aufgesetztem Zielgerät, Fig. 5 eine zweite Ausführungsform des erfindungsgemäßen Implantats, Fig. 6 eine Ansicht des distalen Teils des Implantats der Fig. 5 in auseinander gezogener Darstellung, Fig. 7 eine Darstellung der gegenseitigen Verschiebbarkeit des distalen und des proximalen Teils bei einem Implantat gemäß Fig. 5, Fig. 8 eine Detailansicht der beim Implantat gemäß Fig. 5 vorgesehenen Platte, Fig. 9 eine Ansicht der Unterseite der Platte und Fig. 10 eine Detailansicht des proximalen Teils mit an diesem festgelegter Platte.

In Fig. 1 ist mit 1 ein erfindungsgemäßes Implantat gezeigt mit einem distalen Teil 2 und einem proximalen Teil 3, welche relativ zu einander verschiebbar sind. Der distale Teil 2 und der proximale Teil 3 weisen Bohrungen 4 auf, welche der Aufnahme von Schrauben dienen, um den jeweiligen Implantatteil rotationsgesichert im entsprechenden Knochenteil zu halten. Die Schrauben können dabei so gesetzt werden, dass sie weitere Frakturstellen, beispielsweise solche im Humeruskopf, überbrücken und so Frakturstücke des Humerus festlegen. Der proximale Teil 3 weist an seinem distalen Ende einen Bereich mit vergrößerter Wandstärke auf, welche die Führungsfläche ausbildet, die wiederum mit einer Führungsfläche auf einem stiftartigen Fortsatz 5 des distalen Bereichs zusammenwirkt. Die relative Verschiebung der beiden Teile des Implantats ist somit über diese Führungsflächen geführt, wobei ein Anschlag für die auseinander gezogene Position durch eine verstellbare Schraube 6 im stiftartigen Fortsatz 5 des distalen Teils 2 definiert werden kann.

In Fig. 2 ist ersichtlich, dass in der zusammengesunkenen Position des Implantats der proximale Teil 3 auf einer Schulter 7 des distalen Teils zu liegen kommt, wodurch ein Anschlag für die zusammengesunkene Position definiert ist.

In Fig. 3 ist eine Anzahl von Nuten 8 dargestellt, in welche Stäbe eingesetzt werden können, welche dann, wenn der nicht dargestellte proximale Teil des Implantats ebenfalls geeignete Nuten aufweist, eine Verdrehsicherung der beiden Implantatteile zueinander darstellen.

In Fig. 4 ist mit 9 ein Stift einer Zielvorrichtung bezeichnet, welcher mittels eines Gewindes 10 am proximalen Teil 3 festgelegt ist. Der Stift 9 ist mittels eines Gewindes 11 und einer Mutter 12 beispielsweise unter Zwischenschaltung der eigentlichen Zielvorrichtung 13 gegen eine Hülse 14 verschieb- und verspannbar, sodass eine Fixierung der Position des proximalen Teils 3 zum distalen Teil 2 des Implantats, beim Einsetzen gegeben ist. In der Zielvorrichtung 13 sind Bohrungen 15 vorgesehen, welche mit den Bohrungen 4 des Implantats fluchten, sodass ein durch diese Bohrungen 15 gesetzter Bohrer entsprechende Bohrlöcher für die in die Bohrungen 4 zu setzenden Knochenschrauben herstellen kann.

In Fig. 5 ist eine zweite Ausführungsform des erfindungsgemäßen Implantats dargestellt. Das Implantat besteht wiederum aus einem distalen Teil 16 und einem proximalen Teil 17, die gemäß dem Doppelpfeil 18 in axialer Richtung relativ zueinander frei verschieblich sind. Der distale Teil 16 weist eine Mehrzahl von Bohrungen 19 auf, die der Aufnahme von Fixationsmitteln und insbesondere von Knochenschrauben dienen. Beispielhaft ist eine Knochenschraube 20 dargestellt. Der proximale Teil 17 weist Bohrungen 21, 22 und 23 auf (Fig. 7), die ebenfalls der Aufnahme bzw. der Festlegung von Fixationsmitteln dienen. Im vorliegenden Fall ist eine Platte 24 vorgesehen, die flächig an der Knochenoberfläche aufliegen soll und dadurch die Knochentrümmer stabilisieren soll. Die Platte 24 ist mit Hilfe einer Fixationsschraube 25 und eines Stiftes 26 am proximalen Teil 17 festgelegt. In der Platte 24 sind weiters eine Mehrzahl von Stiften 27 winkelstabil festgelegt, um Knochenfragmente zu stabilisieren. Nähere Details der Ausbildung gemäß Fig. 5 ergeben sich aus der nachfolgenden Beschreibung der Figuren 6 bis 10.

In Fig. 6 ist ersichtlich, dass der distale Teil 16 aus einem Basisteil 28 sowie einem Zwischenstück 29 zusammengesetzt ist. Um eine stabile Verbindung des Basisteils 28 mit dem Zwischenstück 29 zu gewährleisten, weist das Basisteil 28 einen kegelstumpfförmigen Fortsatz 30 auf, der in einen entsprechenden kegelstumpfförmigen Hohlraum des Zwischenstücks 29 im Wesentlichen spielfrei aufgenommen ist. Zur axialen Festlegung des Zwischenstücks 29 auf dem Basisteil 28 ist eine nicht näher dargestellte Befestigungsschraube vorgesehen, die in den hohlen Querschnitt des Zwischenstücks 29 eingeführt wird und in den Fortsatz 30 eingeschraubt wird.

Der zweiteilige Aufbau des distalen Teils 16 ist vorgesehen, um erforderlichenfalls die Festlegung einer Prothese zu erleichtern. Zu diesem Zweck wird beispielsweise das Zwischenstück 29 abgenommen und das Basisteil 28 dient als Prothesenschaft für eine Prothese, wie beispielsweise eine Humeruskopfprothese.

In Fig. 7 ist dargestellt, wie der distale Teil 16 und der proximale Teil 17 relativ zueinander verschiebbar geführt sind. Der distale Teil 16 weist zu diesem Zweck einen zylindrischen Bereich 31 mit verringertem Durchmesser auf, der in einem entsprechenden Hohlraum des proximalen Teils 17 in Richtung des Doppelpfeils 18 geführt ist. Um die Relativdrehung der beiden Teile um die Implantatachse zu verhindern, weist der proximale Teil 17 ein Langloch 32 auf, welches von einem Bolzen 33 durchsetzt ist, der am distalen Teil 16 festgelegt ist. Der Bolzen 33 erlaubt eine axiale Verschiebung der beiden Teile 16 und 17 relativ zueinander, verhindert jedoch eine Drehung der beiden Teile 16 und 17 relativ zueinander um die Implantatachse. Gleichzeitig bildet der Bolzen 13 einen Anschlag zur Begrenzung der Relativverschiebung der beiden Teile 16 und 17 aus.

In Fig. 7 ist weiters ersichtlich, dass der proximale Teil 17 zwei Bohrungen 21 und 22 aufweist, die der Aufnahme von Fixationsmitteln und insbesondere einer Knochenschraube bzw. eines Stiftes dienen, um die Platte 24 am proximalen Teil 17 festzulegen, wie dies in der Folge anhand der Figuren 8 und 10 noch näher erläutert wird. Weiters weist der distale Teil 17 eine weitere Bohrung 23 auf, die quer zu den Bohrungen 21 und 22 verläuft. Die Bohrung 23 dient der Aufnahme einer Knochenschraube 34, mit welcher Knochenfragmente fixiert werden können.

Aus einer Zusammenschau der Fig. 8 mit der Fig. 10 geht hervor, wie die Platte 24 an dem proximalen Teil 17 festgelegt wird. Es ist eine Knochenschraube 25 vorgesehen, die über eine Bohrung in der Platte 24 eingebracht und in ein vorgebohrtes Loch im Knochen eingeschraubt wird, bis sie in der Folge in die Bohrung 21 des proximalen Teils 17 eindringt und dort mit Hilfe ihres Gewindes eingeschraubt wird. Der Schraubenkopf der Knochenschraube 25 bildet einen Anschlag, der mit der Platte 24 zusammenwirkt, sodass die Platte 24 beim Einschrauben der Knochenschraube 25 in das Innengewinde der Bohrung 21 in Richtung zum proximalen Teil 17 gezogen wird. Der Operateur zieht die Platte 24 dabei so lange in Richtung zum distalen Teil 17, bis die Platte 24 am Knochen aufliegt. Der Schraubenkopf der Schraube 25 ist dabei so ausgebildet, dass eine Verschwenkung der Schraube 25 relativ zur Platte 24 ermöglicht wird, sodass sich die Platte 24 exakt an den Knochen anlegen kann.

Die Platte 24 wird in der Folge mit Hilfe des Stiftes 26 gegen Rotation gesichert. Dabei weist der Stift 26 einen Kopf auf, der mit einem Gewinde versehen ist, das mit der entsprechenden Bohrung in der Platte 24 zusammenwirkt, sodass eine winkelstabile Verbindung des Stiftes 26 mit der Platte 24 erreicht wird. Dadurch wird die Platte 24 in der jeweilige Lage fixiert. Der Stift 26 ist weist an seinem Schaft dabei kein Gewinde auf, wodurch ermöglicht wird, dass der Stift 26 zunächst in axialer Richtung in den Knochen eingetrieben wird und in der Folge in die Bohrung 22 im proximalen Teil 17 eindringen kann. Erst wenn der Schraubenkopf die zugehörige Bohrung der Platte 24 erreicht, wird der Stift 26 mit einigen wenigen Umdrehungen in der Platte 24 winkelstabil fixiert. Dadurch, dass der Stift 26 zunächst aber ausschließlich durch Einschlagen in axialer Richtung eingetrieben werden kann, ist ein zielgenaues Einbringen des Stiftes sichergestellt, ohne dass es zu den beim Einschrauben üblicherweise zu beobachtenden Abweichungen von der Einbringungsrichtung ("eiern") kommt.

Die weiteren Fixationsstifte 27 werden in gleicher Weise eingebracht, wie der Stift 26. Der mit einem Gewinde versehene Kopf der Stifte 27 ist dabei derart ausgebildet, dass innerhalb gewisser Grenzen eine Wahl des Winkels zwischen Stift 27 und der Ebene der Platte 24 erfolgen kann. Im gewählten Winkel erfolgt dann eine winkelstabile Fixierung des Stifts 27 in der Platte 24. Damit wird es dem Operateur in einfacher Weise ermöglicht, die Fixationsstifte 27 in einer für die Stabilisierung der einzelnen Knochenfragmente optimalen Richtung einzubringen.

In Fig. 9 ist ersichtlich, dass die dem Knochen zugewandte Fläche der Platte 24 eine Mehrzahl von vorspringenden Spitzen 34 aufweist, die dazu führen, dass die Platte 24 lediglich mit den Spitzen 34 am Knochen aufliegt. Die vorspringenden Spitzen bieten dabei einen guten Halt an den Knochenfragmenten, führen aber gleichzeitig dazu, dass die Platte 24 nicht mit ihrer ganzen Fläche auf dem Knochen aufliegt, sodass Periostkompression vermieden wird.

## Patentansprüche

1. Implantat, insbesondere Marknagel für die Behandlung einer proximalen Humerusfraktur, mit wenigstens einem distalen (2, 16) und wenigstens einem proximalen Teil (3, 17), die zur Veränderung der Implantatlänge axial relativ zueinander verschiebbar angeordnet sind und miteinander zusammenwirkende Führungsflächen aufweisen, **dadurch gekennzeichnet, dass** der proximale (3, 17) und der distale Teil (2, 16) miteinander zusammenwirkende Anschläge zur Begrenzung der axialen Relativverschiebung aufweisen, dass der proximale (3, 17) und der distale Teil (2, 16) innerhalb der durch die Anschläge definierten Begrenzungen nach einer Operation relativ zueinander frei verschiebbar sind, so dass ein Zusammensinken einer subkapitalen Frakturzone innerhalb der durch die Anschläge definierten Grenzen ermöglicht ist, dass der proximale (3, 17) und der distale Teil (2, 16) jeweils mit wenigstens einer Querbohrung (4; 19, 21, 22, 23) zur Aufnahme und/oder Festlegung von Fixationsmitteln versehen sind und dass Mittel zum Verriegeln der Relativverdrehung der beiden Teile (2, 3; 16, 17) um die Implantatachse vorgesehen sind.

2. Implantat nach Anspruch 1, **dadurch gekennzeichnet, dass** als Fixationsmittel wenigstens eine Knochenschraube (19, 25, 34) vorgesehen ist.

3. Implantat nach Ansprüche 1 oder 2 , **dadurch gekennzeichnet, dass** der proximale Teil (3, 17) wenigstens zwei, bevorzugt eine Mehrzahl einander kreuzender Querbohrungen (4; 21, 22, 23) aufweist.

4. Implantat nach Anspruch 1, 2 oder 3, **dadurch gekennzeichnet, dass** die Führungsflächen abschnittsweise zylindrisch ausgebildet sind.

5. Implantat nach einem der Ansprüche 1 bis 4, **dadurch gekennzeichnet, dass** die Führungsflächen jeweils wenigstens eine in axialer Richtung verlaufende Nut (8) aufweisen, in deren gemeinsamem Querschnitt ein Stab aufgenommen ist.

6. Implantat nach einem der Ansprüche 1 bis 5, **dadurch gekennzeichnet, dass** die Führungsflächen einen von einem kreisrunden Querschnitt abweichenden Querschnitt des Implantats bilden.

7. Implantat nach einem der Ansprüche 1 bis 5, **dadurch gekennzeichnet, dass** der proximale (17) oder der distale Teil (16) ein sich in axialer Richtung erstreckendes Langloch (32) aufweist, in dem ein am anderen Teil (16, 17) rotationsfest angeordneter Bolzen (33) in axialer Richtung geführt ist.

8. Implantat nach einem der Ansprüche 1 bis 7, **dadurch gekennzeichnet, dass** als Fixationsmittel eine Platte (24) vorgesehen ist, die relativ zum proximalen Teil (17) des Implantats festlegbar ist, wobei vorzugsweise die Platte (24) eine Mehrzahl von Durchbrechungen zur Aufnahme von Fixationsschrauben (25) und/oder Stiften (26, 27) zum Fixieren von Knochenfragmenten aufweist.

9. Implantat nach Anspruch 8, **dadurch gekennzeichnet, dass** zur Festlegung der Platte (24) wenigstens eine Schraube (25) vorgesehen ist, welche die Platte (24) mit dem proximalen Teil (17) des Implantats verbindet.

10. Implantat nach Anspruch 9, **dadurch gekennzeichnet, dass** zusätzlich zur Schraube (25) ein Stift (26) zum Verbinden der Platte (24) mit dem Implantat vorgesehen ist, dessen Schraubenkopf ein Gewinde zum Einschrauben in die Platte (24) aufweist und dessen dem Schraubenkopf abgewandtes Ende in eine vorzugsweise gewindelose Querbohrung (22) des Implantats einführbar ist.

11. Implantat nach einem der Ansprüche 8 bis 10, **dadurch gekennzeichnet, dass** zum Fixieren von Knochenfragmenten Stifte (27) und/oder Fixationsschrauben vorgesehen sind, die winkelstabil in der Platte (24) festlegbar sind.

12. Implantat nach einem der Ansprüche 1 bis 11 und Zielvorrichtung zum Setzen von Knochenschrauben in Querbohrungen (4) des Implantats, **dadurch gekennzeichnet, dass** die Zielvorrichtung (13) zwei in axialer Richtung relativ zueinander verschiebbare Teile (9, 14) aufweist, von denen der eine Teil (9) mit dem proximalen (3) oder dem distalen (2) Teil des Implantats verbindbar und gemeinsam mit diesem relativ zum anderen Teil (2, 3) verschiebbar ist.

13. Implantat und Zielvorrichtung nach Anspruch 12, **dadurch gekennzeichnet, dass** der eine Teil der Zielvorrichtung (13) von einer über den proximalen Implantatteil (3) schiebbaren Hülse (14) gebildet ist, die sich an einem Vorsprung des distalen Implantatteils (2) abstützt, und der andere Teil der Zielvorrichtung (13) von einem in der Hülse (14) angeordneten Stab (9) gebildet ist, der mit dem proximalen Implantatteil (3) vorzugsweise mit Hilfe einer Schraubverbindung verbindbar ist.

14. Implantat und Zielvorrichtung nach Anspruch 12 oder 13, **dadurch gekennzeichnet, dass** der Stab (9) an seinem proximalen Ende eine Schraubmutter (12) trägt, die sich ggf. mittelbar gegen die Hülse (14) des Zielgeräts stützt.

## Claims

1. Implant, in particular an intramedullary pin for treating a proximal fracture of the humerus, with at least one distal (2, 16) and at least one proximal part (3, 17), which are arranged so as to be displaceable axially relative to each other to modify the length of the implant and have guide surfaces cooperating with each other, **characterized in that** the proximal (3, 17) and the distal part (2, 16) have stops cooperating with each other for limiting the axial relative displacement, that after surgery the proximal (3, 17) and the distal part (2, 16) are freely displaceable relative to each other within the limits defined by the stops, so that it becomes possible for a subcapital fracture zone to sink down within the limits defined by the stops, so that the proximal (3, 17) and the distal part (2, 16) are each provided with at least one transverse bore (4; 19, 21, 22, 23) for receiving and/or fastening fixing means, and that means are provided for locking the relative twisting of the two parts (2, 3; 16, 17) about the implant axis.

2. Implant according to claim 1, **characterized in that** at least one bone screw (19, 25, 34) is provided as fixing means.

3. Implant according to claim 1 or 2, **characterized in that** the proximal part (3, 17) has at least two, preferably a plurality of intersecting transverse bores (4; 21, 22, 23).

4. Implant according to claim 1, 2 or 3, **characterized in that** the guide surfaces are constructed so as to be cylindrical in sections.

5. Implant according to one of claims 1 to 4, **characterized in that** the guide surfaces each have at least one groove (8) running in an axial direction, in the shared cross-section of which a rod is received.

6. Implant according to one of claims 1 to 5, **characterized in that** the guide surfaces form a cross-section of the implant deviating from a circular cross-section.

7. Implant according to one of claims 1 to 5, **characterized in that** the proximal (17) or the distal part (16) has an elongated hole (32) extending in an axial direction, in which a bolt (33), arranged non-rotatably on the other part (16, 17) is guided in an axial direction.

8. Implant according to one of claims 1 to 7, **characterized in that** a plate (24) which is able to be fastened relative to the proximal part (17) of the implant is provided as fixing means, wherein the plate (24) preferably has a plurality of through-holes to receive fixing screws (25) and/or posts (26, 27) for the fixing of bone fragments.

9. Implant according to claim 8, **characterized in that** at least one screw (25) for fastening the plate (24) is provided, which screw connects the plate (24) with the proximal part (17) of the implant.

10. Implant according to claim 9, **characterized in that** in addition to the screw (25), a post (26) is provided for connecting the plate (24) with the implant, the screw head of which post has a thread for screwing into the plate (24) and of which the end farthest from the screw head, is able to be introduced into a preferably threadless transverse bore (22) of the implant.

11. Implant according to one of claims 8 to 10, **characterized in that** posts (27) and/or fixing screws which are able to be fastened in an angle-stable manner in the plate (24) are provided for fixing bone fragments.

12. Implant according to one of claims 1 to 11 and aiming device for placing bone screws in transverse bores (4) of the implant, **characterized in that** the aiming device (13) has two parts (9, 14) which are displaceable relative to each other in an axial direction, of which one part (9) is able to be connected with the proximal (3) or the distal (2) part of the implant and together therewith is able to be displaced relative to the other part (2, 3).

13. Implant and aiming device according to claim 12, **characterized in that** the one part of the aiming device (13) is formed by a sleeve (14) that is able to be pushed over the proximal implant part (3), which sleeve rests on a projection of the distal implant part (2), and the other part of the target device (13) is formed by a rod (9) arranged in the sleeve (14), which rod is able to be connected with the proximal implant part (3) preferably by means of a screw connection.

14. Implant and aiming device according to claim 12 or 13, **characterized in that** a threaded nut (12) is supported on the proximal end of the rod (9), which nut is optionally braced indirectly against the sleeve (14) of the targeting device.

## Revendications

1. Implant, en particulier clou médullaire pour le traitement d'une fracture de l'humérus proximal, comportant au moins une partie distale (2, 16) et au moins une partie proximale (3, 17) qui sont agencées de façon à pouvoir coulisser axialement l'une par rapport à l'autre pour modifier la longueur de l'implant et présentent des surfaces de guidage coopérant les unes avec les autres, **caractérisé en ce que** la partie proximale (3, 17) et la partie distale (2, 16) présentent des butées coopérant les unes avec les autres pour limiter le décalage axial relatif, la partie proximale (3, 17) et la partie distale (2, 16) peuvent coulisser librement l'une par rapport à l'autre dans les limites définies par les butées après une opération, de sorte qu'un affaissement d'une zone de fracture sous-capitale est possible dans les limites définies par les butées, la partie proximale (3, 17) et la partie distale (2, 16) sont dotées respectivement d'au moins un alésage transversal (4, 19, 21, 22, 23) pour recevoir et/ou fixer des moyens de fixation, et des moyens de verrouillage de la rotation relative des deux parties (2, 3, 16, 17) sont prévus autour de l'axe de l'implant.

2. Implant selon la revendication 1, **caractérisé en ce qu'**au moins une vis à os (19, 25, 34) est prévue comme moyen de fixation.

3. Implant selon la revendication 1 ou 2, **caractérisé en ce que** la partie proximale (3, 17) présente au moins deux, de préférence une multiplicité alésages transversaux se croisant (4, 21, 22, 23).

4. Implant selon la revendication 1, 2 ou 3, **caractérisé en ce que** les surfaces de guidage sont formées par endroit de façon cylindrique.

5. Implant selon l'une des revendications 1 à 4, **caractérisé en ce que** les surfaces de guidage présentent respectivement au moins une rainure (8) évoluant dans une direction axiale, dans la section transversale de laquelle une tige est reçue.

6. Implant selon l'une des revendications 1 à 5, **caractérisé en ce que** les surfaces de guidage forment une section transversale de l'implant différente d'une section transversale circulaire.

7. Implant selon l'une des revendications 1 à 5, **caractérisé en ce que** la partie proximale (17) ou la partie distale (16) présente un trou longitudinal (32) s'étendant en direction axiale, dans lequel un boulon (33), disposé sur une autre partie (16, 17) de façon à ne pas pouvoir tourner, est conduit dans une direction axiale.

8. Implant selon l'une des revendications 1 à 7, **caractérisé en ce que**, comme moyen de fixation est prévue une plaque (24) qui peut être fixée par rapport à la partie proximale (17) de l'implant, dans lequel de préférence la plaque (24) présente une multiplicité d'ouvertures pour recevoir des vis de fixation (25) et/ou des broches (26, 27) pour fixer des fragments osseux.

9. Implant selon la revendication 8, **caractérisé en ce que**, pour la fixation de la plaque (24) est prévue au moins une vis (25) qui relie la plaque (24) à la partie proximale (17) de l'implant.

10. Implant selon la revendication 9, **caractérisé en ce qu'**en plus de la vis (25), une broche (26) est prévue pour relier la plaque (24) avec l'implant, dont la tête de vis présente un filetage pour vissage dans la plaque (24) et dont l'extrémité détournée de la tête de vis peut être insérée dans un alésage transversal (22) de préférence sans filetage.

11. Implant selon l'une des revendications 8 à 10, **caractérisé en ce que**, pour la fixation de fragments osseux, des broches (27) et/ou des vis de fixation sont prévues, qui peuvent être fixées avec une stabilité angulaire dans la plaque (24).

12. Implant selon l'une des revendications 1 à 11 et dispositif de visée pour placer des vis à os dans des alésages transversaux (4) de l'implant, **caractérisé en ce que** le dispositif de visée (13) présente deux parties (9, 14) pouvant coulisser l'une par rapport à l'autre en direction axiale, desquelles l'une partie (9) peut être reliée avec la partie proximale (3) ou la partie distale (2) de l'implant et peut coulisser conjointement avec celle-ci par rapport à l'autre partie (2, 3).

13. Implant et dispositif de visée selon la revendication 12, **caractérisé en ce que** l'une partie du dispositif de visée (13) est formée par un manchon (14) pouvant coulisser sur la partie d'implant proximale (3) qui s'appuie sur une protubérance de la partie d'implant distal (2) et l'autre partie du dispositif de visée (13) est formée d'une tige (9) disposée dans le manchon (14) qui peut être reliée à la partie d'implant proximale (3) de préférence à l'aide d'une liaison à vis.

14. Implant et dispositif de visée selon la revendication 12 ou 13, **caractérisé en ce que** la tige (9) porte, sur son extrémité proximale, un écrou à visser (12) qui s'appuie éventuellement indirectement contre le manchon (14) du dispositif de visée.
